# EUROPEAN PATENT SPECIFICATION

(11) **EP 0 377 756 B1**
(45) Date of publication and mention of the grant of the patent: **03.03.1993**
(21) Application number: 89907832.3
(22) Date of filing: 29.06.1989
(51) Int. Cl.: F24F 1/00

(54) **RADIANT LOCAL ROOM COOLING MACHINE**
ZIMMERKÜHLVORRICHTUNG MIT LOKALER STRAHLUNG
REFROIDISSEUR AMBIANT PAR RAYONNEMENT LOCAL

(30) Priority: 30.06.1988 JP 163711/88
(43) Date of publication of application: 18.07.1990
(73) Proprietor: KABUSHIKI KAISHA KOMATSU SEISAKUSHO, Minato-ku Tokyo 107 (JP)
(72) Inventor: KADOTANI, Kanichi, 1200, Manda,Hiratsuka-shi,Kanagawa 254 (JP); ONISHI, Tetsuo, 1200, Manda,Hiratsuka-shi,Kanagawa 254 (JP); ISHIHARA, Takashi, 1200, Manda,Hiratsuka-shi,Kanagawa 254 (JP)
(74) Representative: Meissner, Peter E., Dipl.-Ing.
(86) International application number: PCT/JP89/00649
(87) International publication number: WO 90/00240

(56) References cited:
- EP-A- 0 034 986

## Description

This invention relates to a radiation type local air conditioning unit used in the field of air-conditioning equipment and of office furniture.

Recently, office automation equipment (hereinafter referred to as "OA-equipment") has come into common use in most offices. As the number of OA-equipment units increases, maldistribution of heat load due to the heat generated by them becomes conspicuous, resulting in some parts of the room being rather warm and the other parts thereof too cold. This maldistribution is hard to eliminate by overall air-conditioning, and requires complicated and expensive air-conditioning equipment.
In EP-A-0 034 186 a partition type radiation local unit is published which comprises a cooling unit working by the Peltier effect. The principle of the cooling unit is used invers. The above mentioned working unit recuperates by its cold soldering-spots of a plain the calories from a part of a room in order to restitute the calories in form of radiation by its hot soldering-spots of another plain in the same room. The construction of such a unit is simple, consisting in a plate with a cold and a hot plain. There are not provided any breeze generating means.
The radiation type air conditioners utilizing thermoelectric elements, proposed by the inventors of the present invention (see Japanese Patent Applications No. 62-97606 and 62-160939), are examples of apparatus for locally adjusting room temperature conditions.

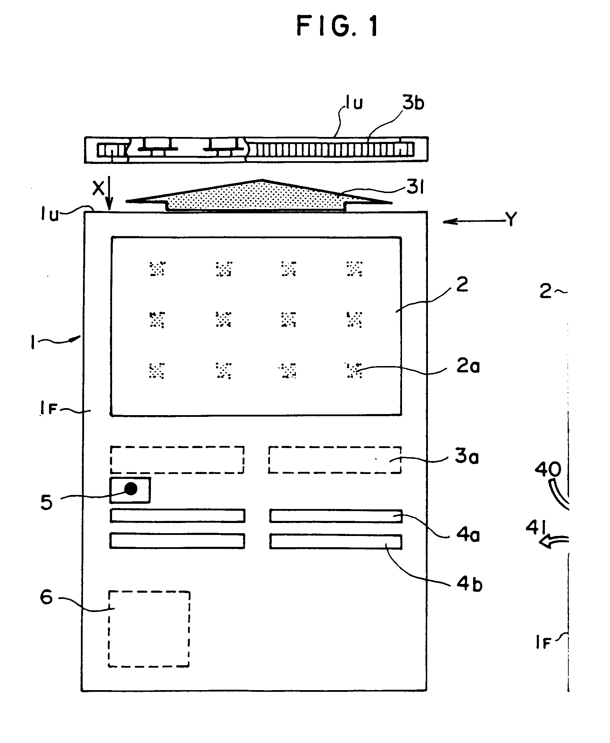

Fig. 1 illustrates a partition unit constituting a radiation air-conditioner of this type; the partition unit is shown here in a front view, a partially sectional view taken in the direction of the arrow X, and a sectional view taken in the direction of the arrow Y. This partition unit is covered on all sides with panels. The front panel 1F has, in the upper section thereof, a cooling plate 2 which is equipped, on its rear side, with a plurality of thermoelectric elements 2a and associated radiation fins 2b. Provided in the middle section of the front panel 1F are air inlets 4a, breeze outlets 4b, and a power switch 5. Heat-dissipating-air inlets 3a and a heat-dissipating-air outlet 3b are provided in the middle section of the rear panel 1R and in the upper panel 1U, respectively. Provided in the middle section of the inner space of the partition unit 1 are a cross-flow fan 3 for heat-dissipating-air blowing and a cross-flow fan 4 for blowing out breeze, which are set in the horizontal position and are spaced apart from each other with respect to a vertical line. A power-source controller 6 is provided below these cross-flow fans.

When, in this construction, the power-source controller 6 is operated by turning on the power switch 5 and electricity is supplied to the thermoelectric elements 2a so as to cause the cooling plate 2 to absorb heat by the Peltier effect, radiation cooling is effected on the front side (the side of the front panel 1F) of the partition unit 1. The air 30, which is sucked in through the heat-dissipating-air inlets 3a by the cross-flow fan 3 for heat-dissipating-air blowing, flows through an air passage 7 and comes into contact with the radiation fins 2b, absorbing the heat which is generated by the thermoelectric elements 2a. The air 30 is then discharged, as exhaust 31, to the exterior through the radiation outlet 3b in the upper panel 1U.

On the other hand, the air 40, which is sucked in through the air inlets 4a in the front panel 1F by the cross-flow fan 4 for blowing out breeze, is blown out, as air 41, through the breeze outlets 4b, thereby effecting convection cooling on the front side (the side of the front panel 1F) of the partition unit 1.

Thus, the radiation cooling by the cooling plate 2 is combined with the convection cooling by the cross-flow fan 4 for blowing out breeze, thereby locally providing comfortable coolness.

However, the above-described partition-type radiation local air-conditioning unit has the following problems:

### 1. Problem regarding coolness

The positions of the breeze outlets in the front panel of the partition unit are not ideal ones from the viewpoint of the synergetic cooling effect to be obtained by radiation cooling and convection cooling, which prevents satisfactory coolness from being obtained.

### 2. Problem regarding the apparatus size

The cross-flow fan for blowing out breeze, set in the horizontal position, occupies a large space in the partition unit, which makes it difficult for the unit size to be diminished. This constitutes a problem since partition-type radiation local air-conditioning units are required to be lighter, smaller and simpler.

It is accordingly an object of this invention to provide a partition-type radiation local air conditioning unit which makes it possible to provide a better feeling of coolness by radiation cooling and to diminish the partition size.

In accordance with this invention, there is provided a partition type radiation local air-conditioning unit comprising: a partition unit; a cooling plate provided in the partition unit; a plurality of thermoelectric elements provided in the cooling plate and adapted to produce the Peltier effect, radiation air-cooling being effected by cooling the cooling plate by the Peltier effect obtained by the thermoelectric elements; and a breeze generating means provided in the partition unit and including a cross-flow fan for blowing out breeze, an air inlet, and a breeze outlet; the breeze generating means being so arranged that the breeze from the breeze outlet is substantially blown against the face of a person sitting in front of the partition unit. Thus, in addition to the radiation cooling by the cooling plate, the breeze from the breeze outlet works effectively, thereby making the person feel a more comfortable coolness.

In the second embodiment of this invention, there is provided ; a plurality of radiation fins associated with thermoelectric elements; a cross-flow fan for heat-dissipating-air blowing provided in the partition unit and adapted to suck in air from the exterior, the air thus sucked in absorbing heat from the radiation fins and being blown out to the exterior as heat-dissipating air; an air passage provided between the cross-flow fan for heat-dissipating-air blowing and the radiation fins; a baffle plate provided in the air passage and dividing it into a heat-dissipating-air passage and a breeze passage; and a breeze outlet provided immediately under the cooling plate and communicating with the breeze passage. The air flowing through the heat-dissipating-air passage absorbs heat from the radiation fins and is blown out to the exterior as heat-dissipating air. The air flowing through the breeze passage is blown out as breeze. This breeze forms a convection cold-air flow together with the cold air descending along the cooling plate, thereby making the person before the unit feel cooler. Furthermore, since no special cross-flow fan is used to generate breeze, the partition unit can be made smaller accordingly.
Fig. 1 is a diagram illustrating a partition unit which constitutes a conventional partition-type radiation local air-conditioning unit;
Fig. 2 is a diagram illustrating a partition unit in accordance with a first embodiment of this invention;
Fig. 3 is a diagram illustrating a partition unit in accordance with a second embodiment of this invention;
Fig. 4 is a diagram illustrating a partition unit in accordance with a third embodiment of this invention;
Fig. 5 is a diagram illustrating a partition unit in accordance with a fourth embodiment of this invention, which is a combination of the embodiments;
   and
Fig. 6 is an overall perspective view of a partition-type radiation local air-conditioning unit in accordance with the fourth embodiment.

Embodiments of this invention will now be described with reference to the accompanying drawings.

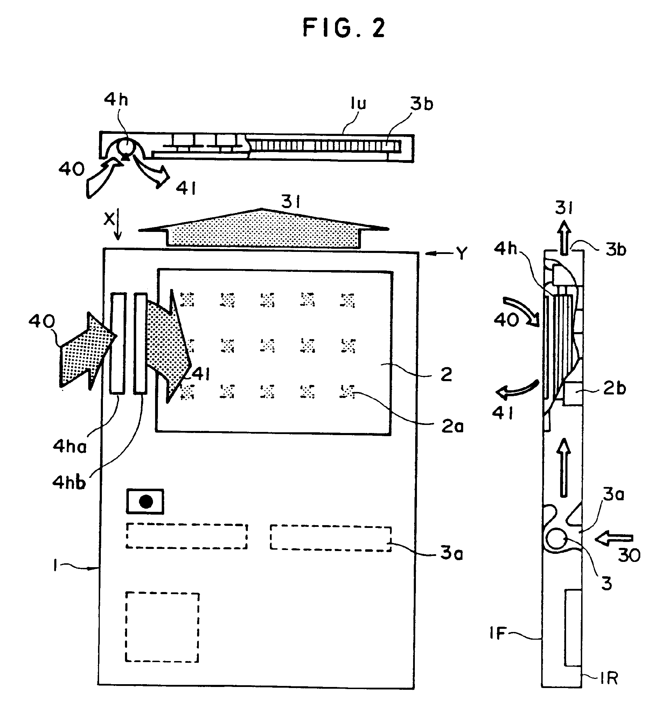

Fig. 2 shows a partition unit in accordance with the first embodiment of this invention, which is claimed in Claim 1; the drawing consists of a front view, a partially sectional view taken in the direction of the arrow X, and a sectional view taken in the direction of the arrow Y. Unlike the above-described conventional air-conditioning unit, this embodiment does not employ a cross-flow fan 4 for blowing out breeze, air inlets 4a, or breeze outlets 4b; in the conventional unit, these components are set in the horizontal position and are arranged below the cooling plate 2. Instead, this embodiment adopts a vertical cross-flow fan 4h, an air inlet 4ha extending in the vertical direction, and a breeze outlet 4hb whose duct angle can be adjusted. These components are arranged in the partition unit, at positions on the left-hand (or right-hand) side (as seen in the drawing) of the cooling plate 2. When the cross-flow fan 4h is driven, the air 40, sucked in through the air inlet 4ha, is blown out through the breeze outlet 4hb, and flows obliquely over the front surface of the cooling plate 2, and is blown, as breeze 41, against the face of a person in front of the partition unit. The air velocity around the human face can be adjusted within the range of about 0 to 2,0m/s. The synergetic effect of this breeze 41 and the radiation cooling by the cooling plate 2 provides a feeling of coolness more effectively.

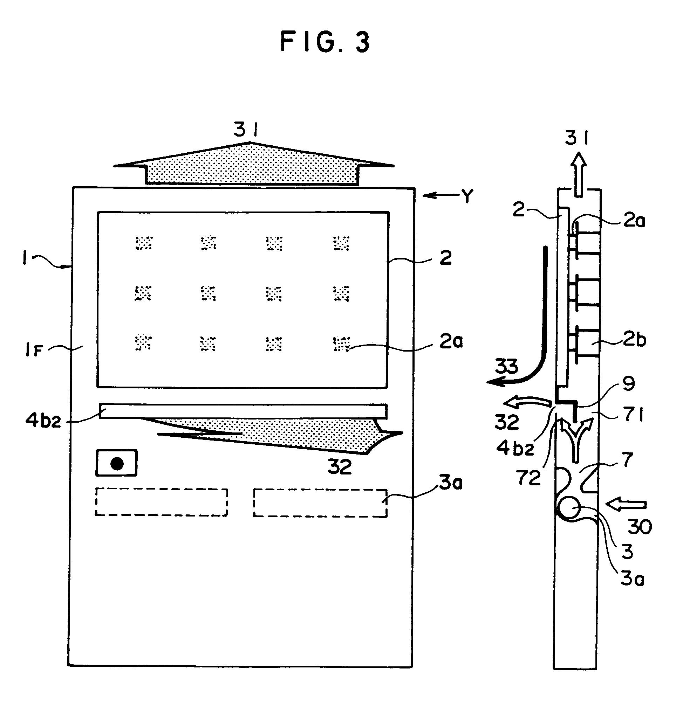

Fig. 3 shows a partition unit in accordance with the second embodiment of this invention; the drawing consists of a front view and a sectional view taken in the direction of the arrow Y. Unlike the above-described conventional air-conditioning unit, this embodiment does not employ a cross-flow fan 4 for blowing out breeze, air inlets 4a, or the breeze outlets 4b; in the conventional unit, these components are set in the horizontal position and are arranged below the cooling plate 2. Instead, this embodiment adopts a baffle plate 9 which is provided in the air passage 7 between the cross-flow fan 3 for heat-dissipating-air blowing and the radiation fins 2b of thermoelectric elements 2a, dividing this air passage 7 into a heat-dissipating-air passage 71 and a breeze-air passage 72. At the same time, a breeze outlet 4b₂ communicating with the breeze air passage 72 is arranged immediately below the cooling plate 2. When the cross-flow fan 3 for heat-dissipating-air blowing is driven, that portion of the air 30 passing through the heat-dissipating-air passage 71 absorbs heat from the radiation fins 2b, and is blown out as heat-dissipating air 31. On the other hand, that portion of the air 30 passing through the breeze air passage 72 flows horizontally under the cooling plate 2 and is blown out through the breeze outlet 4b₂ in such a manner as to be blown, as breeze 32, against the upper part of the body of a person in front of the partition unit (in particular, the shoulders, the face and the head) at a velocity ranging from approximately 0.2 to 0.4m/s. This breeze 32 forms, together with the cold air descending along the cooling plate 2, a convection cold-air flow which works effectively, so that, in addition to the radiation cooling by the cooling plate 2, more coolness can be obtained. Moreover, since no special cross-flow fan 4 or the like has to be provided to obtain breeze, the size of the partition unit 1 can be diminished accordingly.

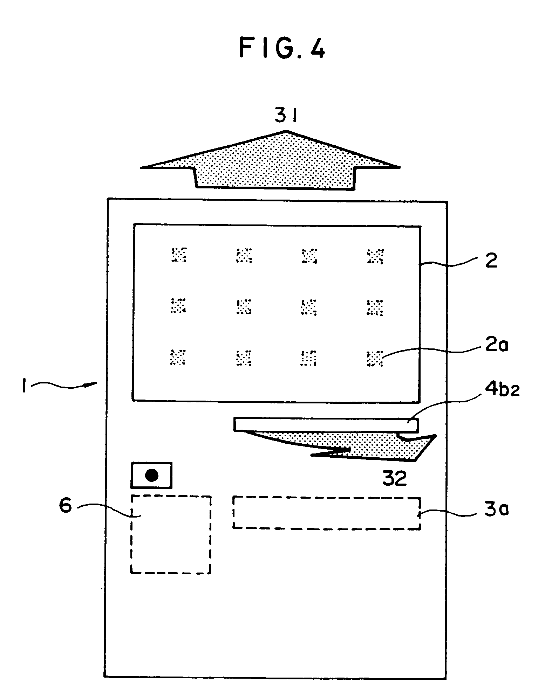

Fig. 4 is a front view of a partition unit in accordance with the third embodiment of this invention, which is claimed in Claim 2. A power-source controller 6 is incorporated into the partition unit 1. At the same time, a breeze outlet 4b₂ whose length is smaller than the width of the cooling plate 2 is arranged immediately below this cooling plate. The length of this outlet can be diminished to half the width of the cooling plate 2, yet it provides substantially the same effect as that of the above-described second embodiment.

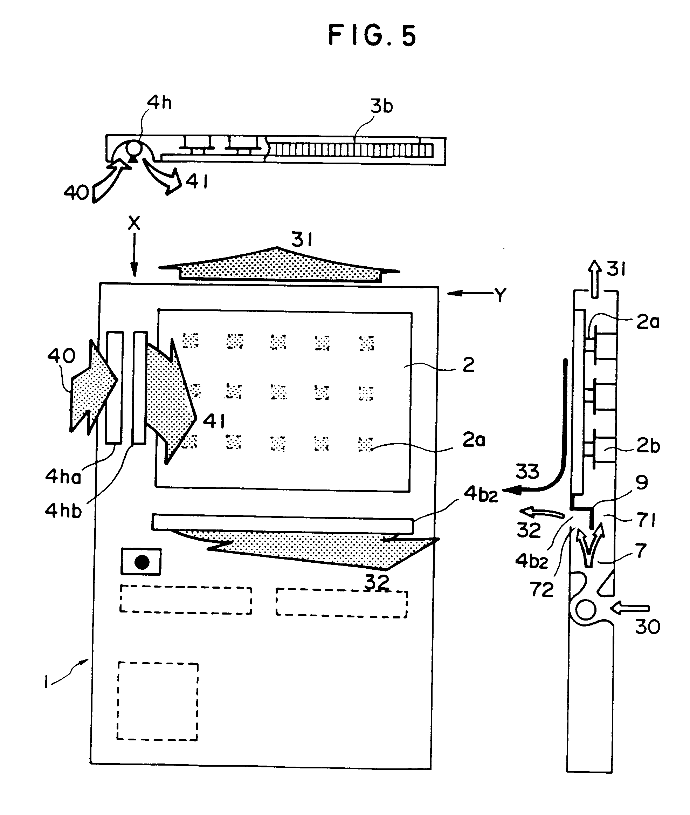

Fig. 5 shows a partition unit in accordance with the fourth embodiment of this invention which consists of a combination of the embodiments claimed in Claims 1 and 2; the drawing consists of a front view, a partially sectional view taken in the direction of the arrow X, and a sectional view taken in the direction of the arrow Y. Arranged in the partition unit at positions on the left-hand (or right-hand) side (as seen in the drawing) of the cooling plate 2 are a vertical cross-flow fan 4h for blowing out breeze, an air inlet 4ha extending in the vertical direction, and a breeze outlet 4hb whose duct angle can be adjusted. At the same time, a baffle plate 9 is provided in the air passage 7 through which flows the air 30 for absorbing heat from the radiation fins 2b of thermoelectric elements 2a. This baffle plate 9 divides the air passage 7 into a heat-dissipating-air passage 71 and a breeze air passage 72. Further, a breeze outlet 4b₂ communicating with the breeze air passage 72 is arranged immediately below the cooling plate 2.

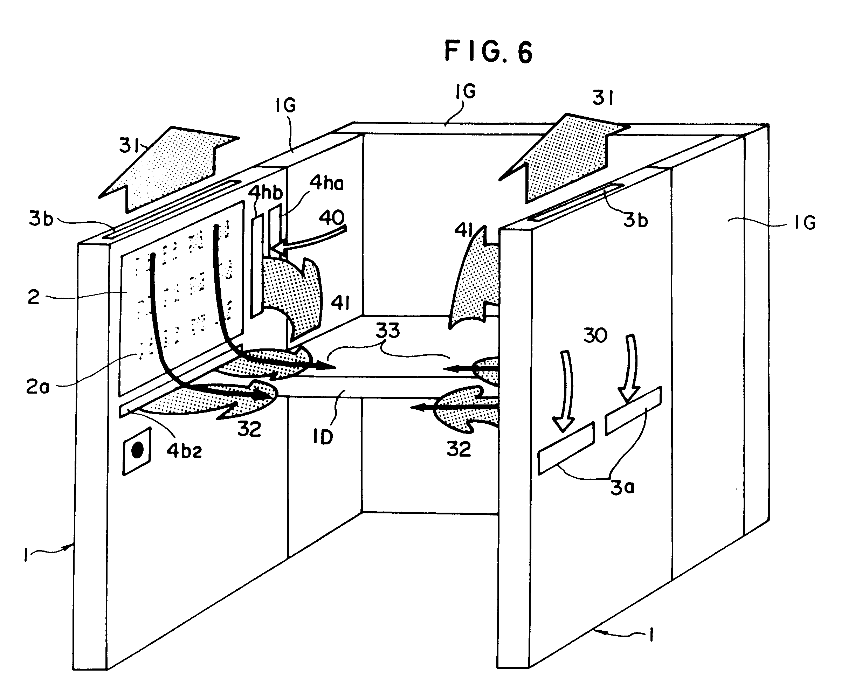

Fig. 6 is an overall perspective view of a partition type radiation local air-conditioning unit which consists of a partition assembly including partition units in accordance with the fourth embodiment of this invention. This radiation type air conditioner is composed of a pair of partition units 1,1 and an ordinary partition 1G arranged between them, the whole exhibiting, in plan, a U-shaped configuration. In one partition unit 1, a vertical cross-flow fan 4h for blowing out breeze, an air inlet 4ha, and a breeze outlet 4hb are arranged on the right-hand side of the cooling plate 2; in the other partition unit 1, these components are arranged on the lefthand side of the cooling plate 2. Arranged in the middle stage of the ordinary partition 1G is a work-table 1D, on which an OA-equipment unit or the like may be set, thus providing a so-called workstation. The operator sitting in front of the work-table 1D receives breeze 41 coming obliquely from the breeze outlets 4hb, the breeze being blown against the face of the operator, making him or her feel coolness. The breeze 32 coming from the breeze outlets 4b₂ provided below the cooling plates 2 is blown, together with the cold air descending along the cooling plates 2, against the upper half of the body of the operator, which also makes him or her feel coolness. In addition, the radiation cooling by the cooling plates 2 makes itself directly felt by the operator. All these cooling effects work as a synergetic effect, thereby further enhancing the coolness.

Thus, in the partition type radiation local air-conditioning unit of this invention, breeze and convection are effectively combined with radiation cooling, thereby making it possible to provide a better feeling of coolness and to diminish the apparatus size.

## Claims

1. A partition type radiation local air-conditioning unit comprising: a partition unit (1); a cooling plate (2) provided in said partition unit; a plurality of thermoelectric elements (2a) provided in said cooling plate and adapted to produce the Peltier effect, radiation air-cooling being effected by cooling said cooling plate by the Peltier effect obtained by said thermoelectric elements; and a breeze generating means provided in said partition unit and including a cross-flow fan (44) for blowing out breeze, an air inlet (44a), and a breeze outlet (44b), said breeze generating means being so arranged that the breeze from said breeze outlet is substantially blown against the face of a person sitting in front of said partition unit.

2. A partition type radiation local air-conditioning unit according to claim 1 characterized in that a plurality of radiation fins (2b) associated with said thermoelectric elements (2a); a cross-flow fan (3) for heat-dissipating-air blowing provided in said partition unit and adapted to suck in air (30) from the exterior, the air thus sucked in absorbing heat from said radiation fins and being blown out to the exterior as heat-dissipating air (31); an air passage (7) provided between said cross-flow fan (3) for heat-dissipating-air blowing and said radiation fins (26); a baffle plate (9) provided in said air passage and dividing said air passage into a heat-dissipating-air passage (71) and a breeze passage (72); and a breeze outlet (4b2) provided immediately below said cooling plate and communicating with said breeze passage.

## Patentansprüche

1. Raumluft-Aufbereitungseinrichtung, bestehend aus folgenden Teilen:
- eine Teileinheit (1),
- eine Kühlplatte (2), die in der Teileinheit (1) vorgesehen ist,
- eine Vielzahl von thermoelektrischen Elementen (2a), die an
der Kühlplatte (2) angebracht sind und nach dem Peltier-Effekt wirken, so daß eine von den Peltier-Elementen erzeugte Kühlung als Strahlungskühlung von der Kühlplatte (2) ausgeht,
- Mittel zur Erzeugung einer Luftströmung, die in der Teileinheit (1) angebracht sind und einen Durchfluß-Lüfter (4h) zur Erzeugung der Ausblas-Luftströmung, einen Lufteinlaß (4ha) und einen Luftauslaß (4hb) einschließen, wobei die Mittel zur Erzeugung der Luftströmung so angeordnet sind, daß die von dem Luftauslaß (4hb) ausgehende Luftströmung im wesentlichen gegen Oberkörper und Gesicht einer vor der Teileinheit (1) sitzenden Person geblasen wird.

2. Einrichtung nach Anspruch 1, dadurch gekennzeichnet, daß eine Vielzahl von Strahlungsrippen (2b) mit den thermoelektrischen Elementen (2a) verbunden sind, daß ein Durchfluß-Lüfter (3) zur Erzeugung einer Warmluftströmung in der Teileinheit (1) vorgesehen ist, der die Umgebungsluft (30) ansaugt, die angesaugte Luft zur Wärmeübernahme an den Strahlungsrippen (2b) vorbeiführt und als Warmluft (31) an die Umgebung wieder ausbläst, daß ein Luftdurchgang (7) zwischen dem Durchfluß-Lüfter (3) zur Warmluftbewegung und den Strahlungsrippen (2b) vorgesehen und innerhalb dessen eine Verteilerplatte (9) angeordnet ist, die den Luftdurchsatz in einen Luftstrom zur Wärmeaufnahme (71) und eine Ausströmung (72) aufteilt und daß eine Ausströmöffnung (4b2) unmittelbar unterhalb der Kühlplatte (2) vorgesehen ist, über die die Ausströmung ausgeleitet wird.

## Revendications

1. Unité de conditionnement d'air locale par rayonnement du type à séparation, comprenant : une unité de séparation (1) ; une plaque de refroidissement (2) prévue dans ladite unité de séparation ; une pluralité d'éléments thermoélectriques (2a) prévus dans ladite plaque de refroidissement et adaptés pour produire l'effet Peltier, le refroidissement d'air par rayonnement étant effectué en refroidissant ladite plaque de refroidissement par l'effet Peltier obtenu par lesdits éléments thermoélectriques ; et des moyens pour engendrer une brise prévus dans ladite unité de séparation et incluant un ventilateur à flux transversal (4h) pour souffler la brise, une entrée d'air (4ha), et une sortie de brise (4hb) ; lesdits moyens pour engendrer une brise étant agencés de sorte que la brise sortant de ladite sortie de brise soit sensiblement soufflée contre le visage d'une personne assise devant ladite unité de séparation.

2. Unité de conditionnement d'air locale par rayonnement du type à séparation selon la revendication 1,
caractérisée par une pluralité d'ailettes de rayonnement (2b) associées auxdits éléments thermoélectriques (2a) ; un ventilateur à flux transversal (3) pour souffler de l'air dissipant la chaleur, prévu dans ladite unité de séparation et adapté pour aspirer de l'air (30) de l'extérieur, l'air ainsi aspiré absorbant de la chaleur à partir desdites ailettes de rayonnement et étant soufflé vers l'extérieur en tant qu'air (31) dissipant la chaleur ; un passage d'air (7) prévu entre ledit ventilateur à flux transversal (3) pour souffler de l'air dissipant la chaleur et lesdites ailettes de rayonnement (2b) ; une plaque-chicane (9) prévue dans ledit passage d'air et divisant ledit passage d'air en un passage d'air dissipant la chaleur (71) et un passage de brise (72) ; et une sortie de brise (4b2) prévue immédiatement au-dessous de ladite plaque de refroidissement et communiquant avec ledit passage de brise.
